(19)

Europäisches Patentamt

European Patent Office

Office européen des brevets

(11) **EP 4 632 679 A1**

(12) **EUROPEAN PATENT APPLICATION**

(43) Date of publication:
**15.10.2025  Bulletin 2025/42**

(21) Application number: **25168351.2**

(22) Date of filing: **03.04.2025**

(51) International Patent Classification (IPC):
***G06T 11/00*** *(2006.01)*

(52) Cooperative Patent Classification (CPC):
**A61B 6/032; G06T 7/0012;** G06T 11/003

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **04.04.2024  JP 2024060581**

(71) Applicant: **Canon Medical Systems Corporation**
**Tochigi 324-0036 (JP)**

(72) Inventors:
- **KIMURA, Asateru**
 **Otawara-shi, 324-0036 (JP)**
- **ARITA, Kosuke**
 **Otawara-shi, 324-0036 (JP)**
- **YAMAZAKI, Yudai**
 **Otawara-shi, 324-0036 (JP)**
- **MURAGUCHI, Yohei**
 **Otawara-shi, 324-0036 (JP)**
- **SASAKI, Sho**
 **Otawara-shi, 324-0036 (JP)**

(74) Representative: **Marks & Clerk LLP**
 **15 Fetter Lane**
 **London EC4A 1BW (GB)**

(54) **X-RAY CT APPARATUS, MEDICAL IMAGE PROCESSING APPARATUS, AND MEDICAL IMAGE PROCESSING METHOD**

(57)    An X-ray CT apparatus (1) according to an embodiment includes a photographing system (19), a generation unit (453), a first calculation unit (455), a specifying unit (457), and a display control unit (458). The generation unit (453) generates X-ray CT image data by performing reconstruction processing on projection data output from the photographing system (19). The first calculation unit (455) calculates an image feature quantity indicating a feature of a relation between one pixel on the X-ray CT image data and one or a plurality of pixels other than the former pixel. The specifying unit (457) specifies a contribution region indicating a region on the X-ray CT image data delineating a feature having a high influence degree for a calculation result of the image feature quantity. The display control unit (458) causes a display apparatus to display the X-ray CT image data representing the contribution region.

FIG.1

**Description**

FIELD

Embodiments described herein relate generally to an X-ray CT apparatus, a medical image processing apparatus, and a medical image processing method.

BACKGROUND

In the related art, a Radiomics feature quantity is known as a feature quantity that is comprehensively extracted from a medical image. Through researches in recent years, for example, it has been found that a texture feature quantity representing texture as one of the Radiomics feature quantities is useful for diagnosis or prognosis prediction.
As an example of such a technique, there is known a technique of inputting a texture feature quantity to a machine learning model that has trained a relation between the texture feature quantity and a diagnostic name (for example, a name of a disease or a symptom) by using a CNN and the like, and estimating the diagnostic name based on an output from the machine learning model.

SUMMARY OF THE INVENTION

According to a first aspect, an X-ray CT apparatus is provided, which includes a photographing system configured to photograph a subject; a generation unit configured to generate X-ray CT image data by performing reconstruction processing on projection data output from the photographing system; a first calculation unit configured to calculate an image feature quantity indicating a feature of a relation between one pixel on the X-ray CT image data and one or a plurality of pixels other than the former pixel; a specifying unit configured to specify a contribution region indicating a region on the X-ray CT image data delineating the feature having a high influence degree for a calculation result of the image feature quantity; and a display control unit configured to cause a display apparatus to display the X-ray CT image data representing the contribution region.
The X-ray CT apparatus may further include a second calculation unit configured to calculate an element feature quantity as a feature quantity corresponding to each of features on the X-ray CT image data for each of the features, and calculate a first influence degree indicating a degree of influence of the feature on a calculation result of the image feature quantity based on the image feature quantity and the element feature quantity. The specifying unit may specify the contribution region based on the first influence degree.
The specifying unit may specify, as the contribution region, a region corresponding to the feature having a highest first influence degree on the X-ray CT image data.
Further, the specifying unit may specify, as the contribution region, a region corresponding to the feature having the first influence degree exceeding a threshold on the X-ray CT image data.
The X-ray CT apparatus may further include an estimating unit configured to estimate a disease condition of the subject based on the image feature quantity and a pre-trained model caused to have a function of outputting an estimation result of a disease condition in response to an input of the image feature quantity by machine learning. The display control unit may cause the display apparatus to display the X-ray CT image data representing the contribution region as grounds for estimation of the estimation result.
The first calculation unit may calculate a plurality of types of the image feature quantities. The second calculation unit may calculate the first influence degree for each of a plurality of types of the image feature quantities.
The X-ray CT apparatus may further include a third calculation unit configured to calculate the first influence degree for each of a plurality of types of the image feature quantities, and calculate, for each pixel on the X-ray CT image data, a second influence degree indicating a degree of influence of the pixel on each of a plurality of types of the image feature quantities based on the first influence degree and a weight indicating how much weight is given to a specific type of the image feature quantity when the pre-trained model outputs the estimation result. The specifying unit may specify the contribution region based on the second influence degree.
The specifying unit may specify, based on the specified contribution region and an image database storing therein a plurality of pieces of the X-ray CT image data representing the contribution region, the X-ray CT image data with similar distribution of contribution regions on the X-ray CT image data in the image database. The display control unit may cause the specified X-ray CT image data to be displayed as a reference image.
According to a second aspect, a medical image processing apparatus is provided, which includes an acquiring unit configured to acquire a medical image; a first calculation unit configured to calculate an image feature quantity indicating a feature of a relation between one pixel on the medical image and one or a plurality of pixels other than the former pixel; a specifying unit configured to specify a contribution region indicating a region on the medical image delineating the feature having a high influence degree for a calculation result of the image feature quantity; and a display control unit configured to cause a display apparatus to display the medical image representing the contribution region.

The medical image processing apparatus may further include a second calculation unit configured to calculate an element feature quantity as a feature quantity corresponding to each of features on the medical image for each of the features, and calculate a first influence degree indicating a degree of influence of the feature on a calculation result of the image feature quantity based on the image feature quantity and the element feature quantity. The specifying unit may specify the contribution region based on the first influence degree.

Further, the specifying unit may specify, as the contribution region, a region corresponding to the feature having a highest first influence degree on the medical image.

The specifying unit may specify, as the contribution region, a region corresponding to the feature having the first influence degree exceeding a threshold on the medical image.

The medical image processing apparatus may further include an estimating unit configured to estimate a disease condition of the subject based on the image feature quantity and a pre-trained model caused to have a function of outputting an estimation result of a disease condition in response to an input of the image feature quantity by machine learning. The display control unit may cause the display apparatus to display the medical image representing the contribution region as grounds for estimation of the estimation result.

The first calculation unit may calculate a plurality of types of the image feature quantities. The second calculation unit may calculate the first influence degree for each of a plurality of types of the image feature quantities. The medical image processing apparatus may further include a third calculation unit configured to calculate, for each pixel on the medical image, a second influence degree indicating a degree of influence of the pixel on each of a plurality of types of the image feature quantities based on the first influence degree and a weight indicating how much weight is given to a specific type of the image feature quantity when the pre-trained model outputs the estimation result. The specifying unit may specify the contribution region based on the second influence degree.

The specifying unit may specify, based on the specified contribution region and an image database storing therein a plurality of the medical images representing the contribution region, the medical image with similar distribution of contribution regions on the medical image in the image database. The display control unit may cause the specified medical image to be displayed as a reference image.

According to a third aspect, a medical image processing method to be performed by a medical image processing apparatus is provided, which includes acquiring a medical image; calculating an image feature quantity indicating a feature of a relation between one pixel on the medical image and one or a plurality of pixels other than the former pixel; specifying a contribution region indicating a region on the medical image delineating a feature having a high influence degree for a calculation result of the image feature quantity; and causing a display apparatus to display the medical image representing the contribution region.

According to a fourth aspect, a storage medium storing a computer-executable program is provided. The program, when executed by a computer, causes the computer to perform acquiring a medical image; calculating an image feature quantity indicating a feature of a relation between one pixel on the medical image and one or a plurality of pixels other than the former pixel; specifying a contribution region indicating a region on the medical image delineating a feature having a high influence degree for a calculation result of the image feature quantity; and causing a display apparatus to display the medical image representing the contribution region.

BRIEF DESCRIPTION OF THE DRAWINGS

FIG. 1 is a block diagram illustrating an example of a configuration of an X-ray CT apparatus according to a first embodiment;
FIG. 2 is a diagram illustrating an example of a shape estimation model according to the first embodiment;
FIG. 3 is a diagram for explaining an example of a calculation method for a texture feature quantity according to the first embodiment;
FIG. 4 is a diagram for explaining another example of the texture feature quantity according to the first embodiment;
FIG. 5 is a diagram illustrating an example of a calculation result of an influence degree according to the first embodiment;
FIG. 6 is a diagram illustrating another example of a calculation result of the influence degree according to the first embodiment;
FIG. 7 is a diagram for explaining an example of X-ray CT image data representing an influence degree for each element according to the first embodiment;
FIG. 8 is a flowchart illustrating an example of processing performed by the X-ray CT apparatus according to the first embodiment;
FIG. 9 is a diagram illustrating an example of the entire configuration of a medical image processing system according to the first embodiment;

FIG. 10 is a flowchart illustrating an example of the entire procedure of processing performed by a medical image processing apparatus according to a second embodiment;

FIG. 11 is a block diagram illustrating an example of a configuration of a medical image processing apparatus according to a first modification;

FIG. 12 is a diagram for explaining an example of a weight of a disease condition estimation model according to the first modification;

FIG. 13 is a diagram for explaining an example of calculation processing for an integrated influence degree according to the first modification;

FIG. 14 is a flowchart illustrating an example of processing performed by the medical image processing apparatus according to the first modification; and

FIG. 15 is a block diagram illustrating an example of the configuration of the medical image processing apparatus according to the first modification.

## DETAILED DESCRIPTION

An X-ray CT apparatus according to an embodiment includes a photographing system configured to photograph a subject, and processing circuitry. The processing circuitry generates X-ray CT image data by performing reconstruction processing on projection data output from the photographing system, calculates an image feature quantity indicating a feature of a relation between one pixel on the X-ray CT image data and one or a plurality of pixels other than the former pixel, specifies a contribution region indicating a region on the X-ray CT image data delineating a feature having a high influence degree for a calculation result of the image feature quantity, and causes a display apparatus to display the X-ray CT image data representing the contribution region.

The following describes embodiments of a medical information processing apparatus and a medical information processing method in detail with reference to the attached drawings.

First embodiment

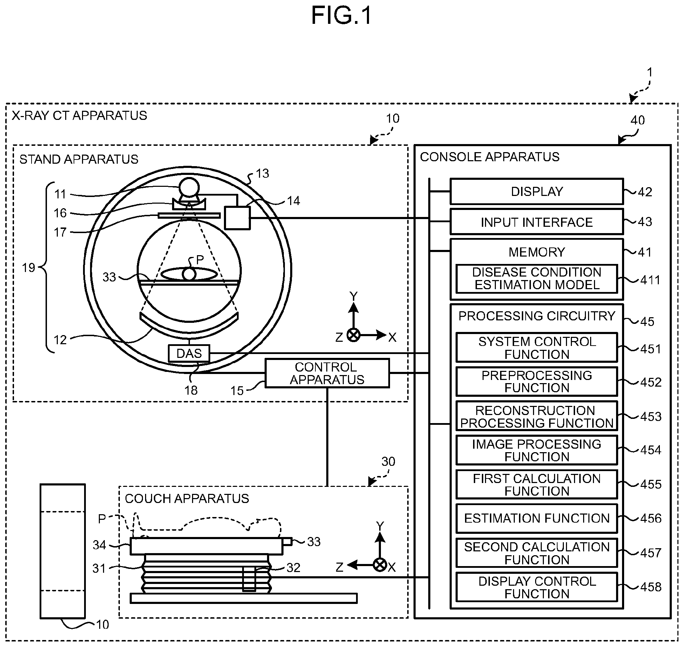

FIG. 1 is a block diagram illustrating an example of a configuration of an X-ray computed tomography (CT) apparatus 1 according to a first embodiment. As illustrated in FIG. 1, the X-ray CT apparatus 1 includes a stand apparatus 10, a couch apparatus 30, and a console apparatus 40.

In the present embodiment, a longitudinal direction of a rotation axis of a rotary frame 13 in a non-tilted state is defined as a Z-axis direction, a direction that is orthogonal to the Z-axis direction and runs from a rotation center toward a pillar supporting the rotary frame 13 is defined as an X-axis, and a direction orthogonal to the Z-axis and the X-axis is defined as a Y-axis.

The stand apparatus 10 includes a photographing system 19 for photographing a medical image used for diagnosis. The photographing system 19 is, for example, constituted of an X-ray tube 11, an X-ray detector 12, a wedge 16, and a collimator 17. That is, the stand apparatus 10 is an apparatus including the photographing system 19 that irradiates a subject P with X-rays, and collects projection data from detection data of X-rays transmitted through the subject P.

The stand apparatus 10 also has an opening part for housing the subject P. A couchtop 33 on which the subject P is placed is housed in the opening part through an entrance on a side where the couch apparatus 30 is disposed.

The stand apparatus 10 includes the X-ray tube 11, the wedge 16, the collimator 17, the X-ray detector 12, an X-ray high-voltage apparatus 14, a data acquisition system (DAS) 18, the rotary frame 13, a control apparatus 15, and the couch apparatus 30.

The X-ray tube 11 is a vacuum tube that emits thermoelectrons from a cathode (filament) toward an anode (target) when high voltage is applied from the X-ray high-voltage apparatus 14. Examples of the X-ray tube 11 include a rotating anode-type X-ray tube that generates X-rays by emitting thermoelectrons to a rotating anode.

The wedge 16 is a filter for adjusting an X-ray dose of X-rays emitted from the X-ray tube 11. Specifically, the wedge 16 is a filter that transmits and attenuates X-rays emitted from the X-ray tube 11 so that distribution of the X-rays emitted from the X-ray tube 11 to the subject P becomes predetermined distribution.

The wedge 16 is, for example, a wedge filter or a bow-tie filter, which is a filter obtained by processing aluminum to have a predetermined target angle and a predetermined thickness.

The collimator 17 is a lead plate and the like for narrowing an irradiation range of X-rays transmitted through the wedge 16, and forms a slit by combining a plurality of lead plates and the like. The collimator 17 may also be referred to as an X-ray aperture.

The X-ray detector 12 detects X-rays that have been emitted from the X-ray tube 11 and passed through the subject P, and outputs an electric signal corresponding to a dose of the X-rays to a data collection apparatus (DAS 18). The X-ray detector 12 includes, for example, a plurality of X-ray detection element arrays in which a plurality of X-ray detection elements are arranged in a channel direction along one arc centered on a focal point of the X-ray tube 11. The channel direction means a circumferential direction of the rotary frame 13.

The X-ray detector 12 includes, for example, the X-ray detection element arrays in which the X-ray detection elements are arranged in the channel direction along one arc centered on the focal point of the X-ray tube 11. The X-ray detector 12 has, for example, a structure in which the X-ray detection element arrays are arranged in a slice direction (also referred to as a body axis direction or a column direction), the X-ray detection element arrays in which the X-ray detection elements are arranged in the channel direction.

The X-ray detector 12 is, for example, a detector of an indirect conversion type including a grid, a scintillator array, and an optical sensor array. The scintillator array includes a plurality of scintillators, and the scintillator includes a scintillator crystal that outputs light with a photon quantity corresponding to an incident X-ray dose. The grid includes an X-ray shielding plate that is disposed on a surface on an X-ray incident side of the scintillator array and has a function of absorbing scattered X-rays.

The optical sensor array has a function of converting light into an electric signal corresponding to an amount of light from the scintillator, and includes, for example, an optical sensor such as a photomultiplier tube (PMT). Alternatively, the X-ray detector 12 may be a detector of a direct conversion type including a semiconductor element that converts incident X-rays into an electric signal.

The X-ray high-voltage apparatus 14 includes electric circuitry such as a transformer and a rectifier, and includes a high-voltage generation apparatus having a function of generating high voltage to be applied to the X-ray tube 11, and an X-ray control apparatus that controls output voltage corresponding to X-rays emitted by the X-ray tube 11. The high-voltage generation apparatus may employ a transformer system, or an inverter system.

The X-ray high-voltage apparatus 14 may be disposed on the rotary frame 13, or may be disposed on a fixed frame (not illustrated) side of the stand apparatus 10. The fixed frame is a frame that supports the rotary frame 13 in a rotatable manner.

The DAS 18 includes an amplifier that performs amplification processing on an electric signal output from each X-ray detection element of the X-ray detector 12, and an A/D converter that converts the electric signal into a digital signal, and generates detection data. The detection data generated by the DAS 18 is transferred to the console apparatus 40. The detection data is, for example, a sinogram.

The sinogram is data indicating projection data that is generated for each position of the X-ray tube 11 (hereinafter, also referred to as a view angle) and for each X-ray detection element in association with a view direction and the channel direction. Herein, the view direction corresponds to the view angle, and means an irradiation direction of X-rays.

In a case of performing a single scan using only one detection element array in the X-ray detector 12, one sinogram can be generated for one scan. In a case of performing a helical scan or a volume scan using a plurality of detection element arrays in the X-ray detector 12, a plurality of sinograms can be generated for one scan.

The rotary frame 13 is a frame having an annular shape that supports the X-ray tube 11 and the X-ray detector 12 to be opposed to each other, and rotates the X-ray tube 11 and the X-ray detector 12 by the control apparatus 15. The rotary frame 13 further supports the X-ray high-voltage apparatus 14 and the DAS 18 in addition to the X-ray tube 11 and the X-ray detector 12.

The rotary frame 13 is supported by a non-rotary portion (for example, a fixed frame, which is not illustrated in FIG. 1) of the stand apparatus in a rotatable manner. A rotation mechanism includes, for example, a motor that generates rotational driving force, and a bearing that transmits the rotational driving force to the rotary frame 13 to be rotated. The motor is, for example, disposed on the non-rotary portion. The bearing is physically connected to the rotary frame 13 and the motor, and the rotary frame 13 rotates in accordance with rotational force of the motor.

Communication circuitry of a non-contact type or a contact type is disposed on each of the rotary frame 13 and the non-rotary portion, which allows communication between a unit supported by the rotary frame 13 and the non-rotary portion or an external apparatus of the stand apparatus 10.

For example, in a case of employing optical communication as a non-contact communication scheme, the detection data generated by the DAS 18 is transmitted from a transmitter including a light emitting diode (LED) disposed on the rotary frame 13 to a receiver including a photodiode disposed on the non-rotary portion of the stand apparatus by optical communication, and is further transferred from the non-rotary portion to the console apparatus 40 by the transmitter.

As the communication scheme, a contact-type data transmission scheme using a slip ring and an electrode brush may also be employed in addition to non-contact type data transmission such as a capacitive coupling type or a radio wave type.

The control apparatus 15 includes processing circuitry including a CPU and the like, and a driving mechanism such as a motor and an actuator. The control apparatus 15 has a function of performing operation control for the stand apparatus 10 and the couch apparatus 30 by receiving an input signal from an input interface 43 (described later) attached to the console apparatus 40 or the stand apparatus 10.

For example, the control apparatus 15 performs, by receiving the input signal, control for rotating the rotary frame 13, control for tilting the stand apparatus 10, and control for causing the couch apparatus 30 and the couchtop 33 to operate. The control for tilting the stand apparatus 10 is implemented by rotating the rotary frame 13 about an axis parallel with the X-axis direction by the control apparatus 15 based on inclination angle (tilt angle) information input through the input interface attached to the stand apparatus 10.

The control apparatus 15 may be disposed on the stand apparatus 10, or may be disposed on the console apparatus 40.

The couch apparatus 30 is an apparatus for placing and moving the subject P as a scan target, and includes a base 31, a couch driving apparatus 32, the couchtop 33, and a support frame 34. The base 31 is a housing that supports the support frame 34 to be movable in a vertical direction. The couch driving apparatus 32 is a motor or an actuator that moves the couchtop 33 on which the subject P is placed in a major axis direction thereof (Z-axis direction in FIG. 1).

The couchtop 33 disposed on an upper surface of the support frame 34 is a plate on which the subject P is placed. The couch driving apparatus 32 may move, in addition to the couchtop 33, the support frame 34 in the major axis direction of the couchtop 33.

The couch driving apparatus 32 moves the base 31 in an upper-and-lower direction in accordance with a control signal from the control apparatus 15. The couch driving apparatus 32 also moves the couchtop 33 in the major axis direction (Z-axis direction) in accordance with a control signal from the control apparatus 15.

The console apparatus 40 is an apparatus that receives an operation on the X-ray CT apparatus 1 by an operator, and reconstructs X-ray CT image data from X-ray detection data collected by the stand apparatus 10. The console apparatus 40 includes a memory 41, a display 42, the input interface 43, and processing circuitry 45.

For example, the memory 41 is implemented by a random access memory (RAM), a semiconductor memory element such as a flash memory, a hard disk, an optical disc, and the like.

The memory 41 stores therein, for example, projection data and reconstructed image data. The memory 41 also stores therein a photographing protocol.

Herein, the photographing protocol specifies a procedure and the like for photographing the subject P to acquire an image by controlling the photographing system 19. The photographing protocol is, for example, a group of parameters such as a photographing part, a photographing condition, a photographing range, a reconstruction condition, an operation of the stand apparatus 10 (photographing system 19), and an operation of the couch apparatus 30.

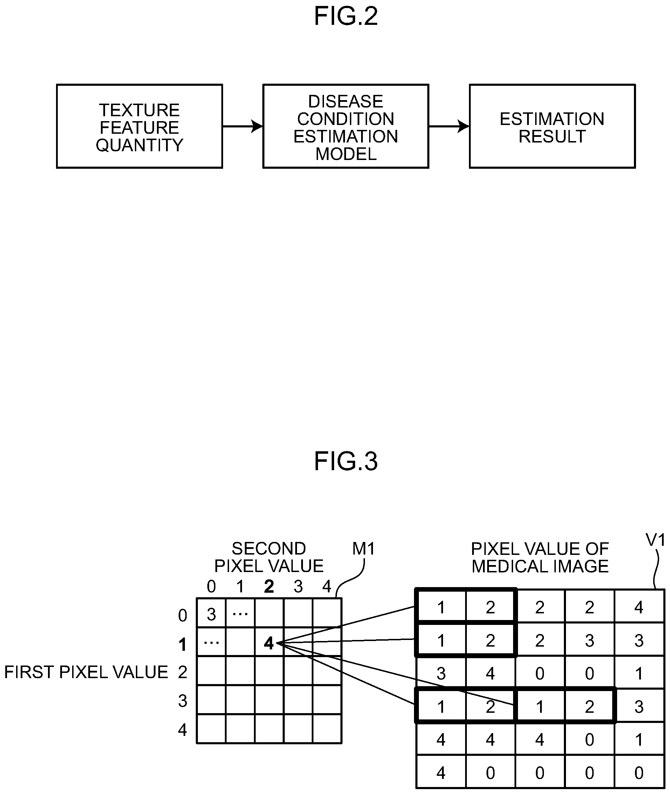

The memory 41 also stores therein a disease condition estimation model 411. The disease condition estimation model 411 is a pre-trained model generated by a known machine learning (including deep learning) technique using a texture feature quantity and information representing a disease condition (for example, a name of a disease and a stage of a disease) as data for training. The following describes the disease condition estimation model 411 with reference to FIG. 2. FIG. 2 is a diagram illustrating an example of the disease condition estimation model 411.

As illustrated in FIG. 2, the disease condition estimation model 411 is a pre-trained model that outputs an estimation result of a disease condition in response to an input of a texture feature quantity. The estimation result of the disease condition represents, for example, an estimated disease condition and a probability of the disease condition in writing such as "This tumor has a 90% probability of glioma grade 4".

As the disease condition estimation model 411, a plurality of models may be stored corresponding to target organs. The texture feature quantity input to the disease condition estimation model 411 may be feature quantities of a plurality of types of texture features. There may be a plurality of estimated symptoms output to the disease condition estimation model 411. In this case, the disease condition estimation model 411 may output, for each of the symptoms, a probability of being the symptom so that the sum total of the probability becomes 100%.

The memory 41 also stores therein a dedicated computer program for implementing a system control function 451, a preprocessing function 452, a reconstruction processing function 453, an image processing function 454, a first calculation function 455, an estimation function 456, a second calculation function 457, and a display control function 458 (described later).

The display 42 is a monitor that the operator refers to, and displays various pieces of information. For example, the display 42 outputs a medical image (CT image) generated by the processing circuitry 45, a graphical user interface (GUI) for receiving various operations from the operator, and the like. For example, the display 42 is a liquid crystal display or a cathode ray tube (CRT) display.

Returning to FIG. 1, the description will be continued.

The input interface 43 receives various input operations from the operator, and converts the received input operations into electric signals to be output to the processing circuitry 45. For example, the input interface 43 receives, from the operator, a collection condition for collecting projection data, a reconstruction condition for reconstructing a CT image, an image processing condition for generating a postprocessed image from the CT image, and the like.

For example, the input interface 43 is implemented by a mouse, a keyboard, a trackball, a switch, a button, a joystick, and the like. The input interface 43 may be disposed on the stand apparatus 10. The input interface 43 may also be constituted of a tablet terminal and the like that can wirelessly communicate with a main body of the console apparatus 40.

The processing circuitry 45 controls an operation of the entire X-ray CT apparatus 1. The processing circuitry 45 includes, for example, the system control function 451, the preprocessing function 452, the reconstruction processing function 453, the image processing function 454, the first calculation function 455, the estimation function 456, the second calculation function 457, and the display control function 458.

The reconstruction processing function 453 is an example of a generation unit. The first calculation function 455 is an example of a first calculation unit and a second calculation unit. The estimation function 456 is an example of an estimating unit. The second calculation function 457 is an example of a second calculation unit and a specifying unit. The display control function 458 is an example of a display control unit.

In the embodiment, respective processing functions executed by the system control function 451, the preprocessing function 452, the reconstruction processing function 453, the image processing function 454, the first calculation function 455, the estimation function 456, the second calculation function 457, and the display control function 458 as constituent elements are stored in the memory 41 in a form of a computer-executable computer program. The processing circuitry 45 is a processor that implements a function corresponding to each computer program by reading out, from the memory 41, and executing the computer program.

In other words, the processing circuitry 45 after reading out each computer program has each function illustrated in the processing circuitry 45 in FIG. 1.

In FIG. 1, the processing functions executed by the system control function 451, the preprocessing function 452, the reconstruction processing function 453, the image processing function 454, the first calculation function 455, the estimation function 456, the second calculation function 457, and the display control function 458 are assumed to be implemented by the single processing circuitry 45, but the processing circuitry 45 may be configured by combining a plurality of independent processors, and the functions may be implemented by executing computer programs by the respective processors.

In other words, each of the functions described above may be configured as a computer program and each of the computer programs may be executed by one piece of the processing circuitry, or a specific function may be implemented on dedicated independent computer program execution circuitry.

The system control function 451 controls various functions of the processing circuitry 45 based on an input operation received from the operator via the input interface 43. For example, the system control function 451 receives inputs of user information for login (for example, a user ID and the like), subject information, and the like via the input interface 43. For example, the system control function 451 receives an input of a photographing protocol via the input interface 43.

The preprocessing function 452 generates data obtained by performing preprocessing such as logarithm conversion processing, offset processing, sensitivity correction processing between channels, and beam hardening correction on the detection data output from the DAS 18. Data before the preprocessing (detection data) and data after the preprocessing may be collectively referred to as projection data.

The reconstruction processing function 453 generates a plurality of pieces of slice image data by performing reconstruction processing on the projection data generated by the preprocessing function 452 using a filtered back projection method, a successive approximation reconstruction method, or the like in accordance with the reconstruction condition. The data before the preprocessing (the detection data and the data after the preprocessing) may be collectively referred to as projection data.

The image processing function 454 generates postprocessed image data obtained by performing postprocessing on the pieces of slice image data generated by the reconstruction processing function 453. Data before the postprocessing (the pieces of slice image data) and data after the postprocessing may be collectively referred to as X-ray CT image data.

Herein, the postprocessing is a concept indicating processing on the pieces of slice image data generated by the reconstruction processing function 453. For example, the postprocessing is processing including noise elimination, multi-planar reconstruction (MPR) display of the pieces of slice image data, rendering of volume data, and the like.

For example, the image processing function 454 converts the X-ray CT image data generated by the reconstruction processing function 453 into tomogram data of an optionally cross section or three-dimensional image data using a well-known method based on an input operation received from the operator via the input interface 43. The three-dimensional image data may be directly generated by the reconstruction processing function 453.

The first calculation function 455 calculates a texture feature quantity as one of a Radiomics feature quantity. The texture feature quantity is an example of an image feature quantity. The present embodiment describes an example of using the texture feature quantity as the image feature quantity, but the image feature quantity is not limited to the texture feature quantity. It is sufficient that the image feature quantity is a feature quantity related to a feature representing a relation between a certain pixel and pixels other than the certain pixel.

For example, the first calculation function 455 calculates a texture feature quantity of the X-ray CT image data postprocessed by the image processing function 454 in accordance with a calculation method that is defined for each type of the texture feature quantity stored in the memory 41.

By way of example, the first calculation function 455 calculates a Joint Energy feature quantity as one of the texture feature quantities calculated by texture analysis using a gray-level co-occurrence matrix (GLCM).

The Joint Energy feature quantity is calculated by the following expression (1).

$$joint\ energy = \sum_{i=1}^{N_g} \sum_{j=1}^{N_g} \left(p(i,j)\right)^2 \qquad \cdots (1)$$

In the expression (1), $N_g$ represents a maximum value of a pixel value in the X-ray CT image data. The Joint Energy feature quantity can be obtained by creating an appearance probability map of a combination of adjacent pixel values, calculating feature quantities for respective combinations of adjacent pixel values in the X-ray CT image data, and totaling the feature quantities.

The following describes a calculation method for the Joint Energy feature quantity with reference to FIG. 3. FIG. 3 is a diagram for explaining an example of the calculation method for the texture feature quantity according to the embodiment.

FIG. 3 is a diagram representing a relation between a table V1 representing a pixel value of each pixel of the X-ray CT image data and a map M1 as a map of an appearance probability of a combination of adjacent pixel values. The map M1 represents arrangement of adjacent pixel values in a lateral (row) direction, and the number of appearances of combinations of the adjacent pixel values on the X-ray CT image data.

In the example of FIG. 3, bold-faced portions in the map M1 represent that there are four combinations of pixels that are adjacent to each other in order of a pixel having a pixel value of "1" and a pixel having a pixel value of "2" on the table V1. In this way, the first calculation function 455 creates the map M1 by counting the number of appearances of each element of the Joint Energy feature quantity for each combination of adjacent pixel values (hereinafter, also referred to as an element of the Joint Energy feature quantity).

Next, the first calculation function 455 calculates an element feature quantity of the Joint Energy feature quantity, which is a feature quantity of each element of the Joint Energy feature quantity, in accordance with the created map M1. The first calculation function 455 calculates the Joint Energy feature quantity by totaling calculated element feature quantities of the Joint Energy feature quantity. Specifically, the first calculation function 455 calculates the Joint Energy feature quantity as represented by the following expression (2).

$$p(0,0)^2 + p(0,1)^2 + \cdots + p(4,4)^2 = 300 \qquad \cdots (2)$$

In the expression (2), $p(0, 0)^2$ represents an element feature quantity of the Joint Energy feature quantity of an element of the Joint Energy feature quantity in which pixels are adjacent to each other in order of a pixel having a pixel value of "0" and a pixel having a pixel value of "0". In the example of the expression (2), the first calculation function 455 calculates the Joint Energy feature quantity to be 300 as a total value of the element feature quantity of the Joint Energy feature quantity.

As another example, the first calculation function 455 calculates a small area emphasis (SAE) feature quantity as one of the texture feature quantities calculated by texture analysis using a matrix (gray level size zone matrix: GLSZM) that is obtained by counting the number of groups of connected pixels.

The SAE feature quantity is calculated by the following expression (3).

$$SAE = \frac{\sum_{i=1}^{N_g} \sum_{j=1}^{N_s} \frac{P(i,j)}{j^2}}{N_z} \qquad \cdots (3)$$

In the expression (3), $N_g$ represents a maximum value of a pixel value in the X-ray CT image data. Additionally, $N_s$ represents the number of maximum connected components in the X-ray CT image data. The SAE feature quantity can be obtained by creating an appearance probability map of maximum connected components of pixel values, calculating feature quantities for the respective maximum connected components of the pixel values in the X-ray CT image data, and totaling the feature quantities.

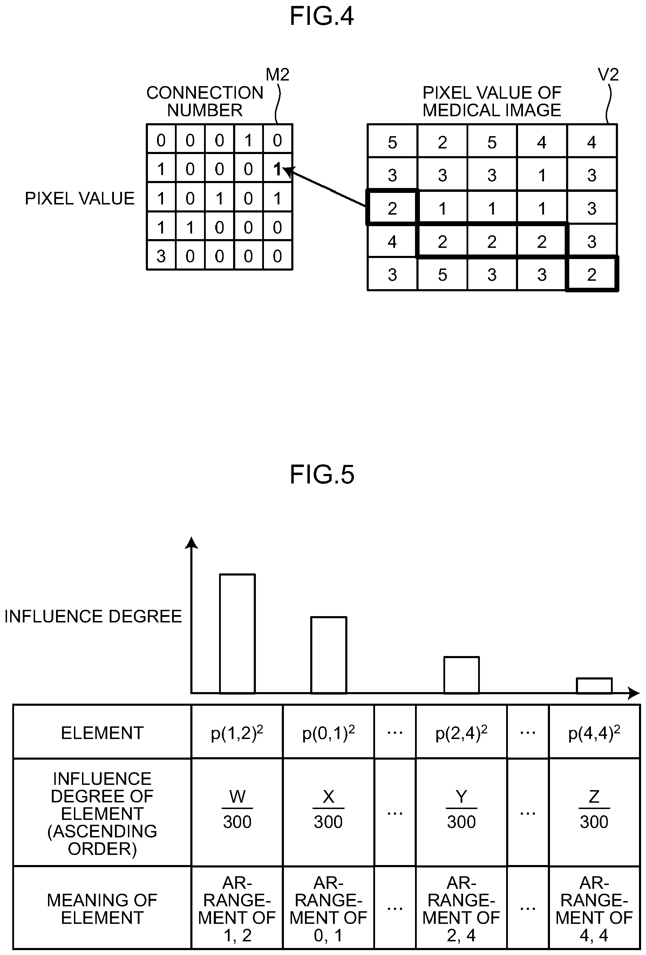

The following describes a calculation method for the SAE feature quantity with reference to FIG. 4. FIG. 4 is a diagram for explaining another example of the calculation method for the texture feature quantity according to the embodiment.

FIG. 4 is a diagram representing a relation between a table V2 representing a pixel value of each pixel of the X-ray CT image data and a map M2 as an appearance probability map of the maximum connected components of the pixel values. The map M2 represents, for each pixel value, the number of appearances of a connection number representing the number of connected pixels each having that pixel value.

In the example of FIG. 4, a bold-faced portion in the map M2 represents that there is one region in which five pixels having a pixel value of "2" are connected on the table V2. In this way, the first calculation function 455 creates the map M2 by counting the number of appearances of each elements of the SAE for each connection number of each pixel value (hereinafter, also referred to as an element of the SAE feature quantity).

Subsequently, the first calculation function 455 calculates an element feature quantity of the SAE feature quantity, which is a feature quantity of each element of the SAE feature quantity, in accordance with the created map M2. The first calculation function 455 calculates the SAE feature quantity by totaling calculated element feature quantities of the SAE feature quantity. Specifically, the SAE feature quantity is calculated as represented by the following expression (4).

$$\frac{P(1,1)}{1^2} + \frac{P(1,2)}{2^2} + \cdots + \frac{P(5,5)}{5^2} = 100 \qquad \cdots (4)$$

In the expression (4), $p(1,1)/1^2$ represents an element feature quantity of the SAE feature quantity of an element of the SAE feature quantity in which "1" pixel having a pixel value of "1" is connected. In the example of the expression (4), the first calculation function 455 calculates the SAE feature quantity to be 100 as a total value of the element feature quantity of the SAE feature quantity.

Returning to FIG. 1, the description will be continued. The estimation function 456 estimates a disease condition of the subject. For example, the estimation function 456 inputs the texture feature quantity calculated by the first calculation function 455 to the disease condition estimation model 411 stored in the memory 41. The estimation function 456 estimates the disease condition of the subject based on an output of the disease condition estimation model 411.

By way of example, in a case in which the output of the disease condition estimation model 411 is "This tumor has a 90% probability of glioma grade 4", the estimation function 456 causes "This tumor has a 90% probability of glioma grade 4" to be an estimation result.

In a case in which the disease condition estimation model 411 outputs, for each of a plurality of symptoms, a probability of being the symptom so that the sum total of probabilities becomes 100%, the estimation function 456 may cause only a symptom the probability of which exceeds a threshold to be the estimation result.

In a case in which a plurality of models are stored as disease condition estimation models 411 corresponding to target organs, the estimation function 456 may detect an organ delineated in the X-ray CT image data using a well-known image recognition technique and the like, and estimate the disease condition using a model corresponding to the organ.

The second calculation function 457 calculates, for each element, an influence degree indicating a degree of influence on a calculation result of the texture feature quantity. In this case, the influence degree is an example of a first influence degree.

For example, the second calculation function 457 calculates the influence degree for each element based on the texture feature quantity calculated by the first calculation function 455 and the element feature quantity of each element of the texture feature quantity. Specifically, the second calculation function 457 calculates the influence degree for each element by dividing each element feature quantity by the texture feature quantity.

The second calculation function 457 may specify a contribution region indicating a region on the X-ray CT image data delineating a feature having a high influence degree for a calculation result of the texture feature quantity.

For example, the second calculation function 457 may sort calculated influence degrees in ascending order, and specify, as the contribution region, a region on the X-ray CT image data corresponding to an element the calculated influence degree of which is the highest. Alternatively, for example, the second calculation function 457 may specify, as the contribution region, a region corresponding to a feature having the influence degree exceeding a threshold.

FIG. 5 is a diagram for explaining an example of a calculation result of the influence degree. In FIG. 5, the influence degrees of the elements are sorted in ascending order for the Joint Energy feature quantity calculated by the expression (2) and illustrated in FIG. 3.

The example of FIG. 3 and the expression (2) represents the fact that an influence degree of an element of $p(1,2)^2$ is W/300, which is the highest. The element of $p(1,2)^2$ represents a region in which a pixel having a pixel value of "1" and a pixel having a pixel value of "2" are adjacent to each other in order in the lateral direction on the X-ray CT image data.

Similarly, the example of FIG. 3 and the expression (2) represents that an influence degree of an element of $p(4,4)^2$ is Z/300, which is the lowest. The element of $p(4,4)^2$ represents a region in which a pixel having a pixel value of "4" and a pixel having a pixel value of "4" are adjacent to each other in order in the lateral direction on the X-ray CT image data.

In a case of specifying the contribution region in the example of FIG. 3 and the expression (2), the second calculation function 457 specifies, as the contribution region, a region on the X-ray CT image data corresponding to the element of $p(1, 2)^2$ the influence degree of which is the highest.

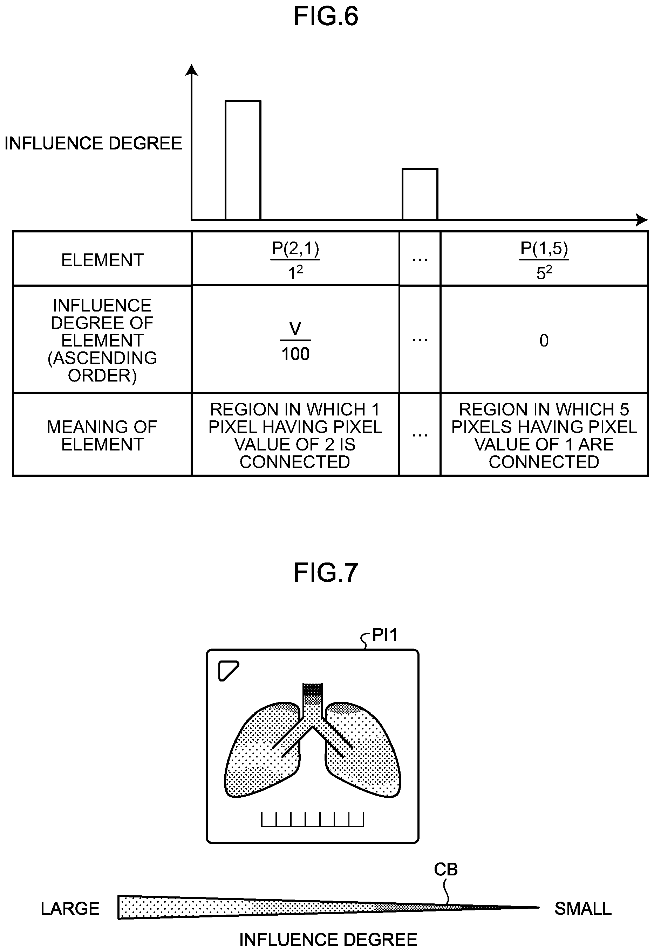

FIG. 6 is a diagram for explaining another example of the calculation result of the influence degree. In FIG. 6, influence degrees of the element feature quantities are sorted in ascending order for the SAE feature quantity calculated by the expression (4) and illustrated in FIG. 4.

The example of FIG. 4 and the expression (4) represents the fact that an influence degree of an element of $P(2, 1)/1^2$ is V/100, which is the highest. The element of $P(2, 1)/1^2$ represents a region in which "1" pixel having a pixel value of "2" is connected on the X-ray CT image data.

Similarly, the example of FIG. 4 and the expression (4) represents the fact that an influence degree of an element of $P(1, 5)/5^2$ is 0, which is the lowest. The element of $P(1, 5)/5^2$ represents a region in which "5" pixels having a pixel value of "1" are connected on the X-ray CT image data.

In a case of specifying the contribution region in the example of FIG. 4 and the expression (4), the second calculation function 457 specifies, as the contribution region, a region on the X-ray CT image data corresponding to the element of $P(1, 5)/5^2$ the influence degree of which is the highest.

In a case in which a ratio of a total area of the contribution region specified by the second calculation function 457 to an area of the entire X-ray CT image data is smaller than a predetermined threshold, the system control function 451 may scan the subject again (re-emission of X-rays to the subject).

Returning to FIG. 1, the description will be continued. The display control function 458 causes the display apparatus to display the X-ray CT image data representing the influence degree of each element.

For example, in a case of receiving an instruction to display grounds for estimation of the disease condition from the user, the display control function 458 causes the display 42 to display the X-ray CT image data representing the influence degree of each element by changing a display color.

By way of example, in accordance with the calculation result of the influence degree obtained by the second calculation function 457, the display control function 458 performs processing of changing the display color of each pixel of the X-ray CT image data in a stepwise manner so that a region having the highest influence degree is red, a region corresponding to a median of the influence degree is yellow, and a region having the lowest influence degree is blue.

In the example of FIG. 3 and the expression (2), the display control function 458 causes the display 42 to display the X-ray CT image data in which the display color of each pixel is changed in a stepwise manner so that a region corresponding to the element of $p(1, 2)^2$ on the X-ray CT image data is red, and a region corresponding to the element of $p(4, 4)^2$ is blue.

In the example of FIG. 4 and the expression (4), the display control function 458 causes the display 42 to display the X-ray CT image data in which the display color of each pixel is changed in a stepwise manner so that a region corresponding to the element of $P(2, 1)/1^2$ on the X-ray CT image data is red, and a region corresponding to the element of $P(1, 5) /5^2$ is blue.

FIG. 7 is a diagram for explaining an example of display of the grounds for estimation. As illustrated in FIG. 7, a processed image PI1 and a color bar CB are displayed on a display screen of the grounds for estimation. The processed image PI1 is the X-ray CT image data in which the display color of each pixel is changed in accordance with the influence degree calculated by the second calculation function 457. The color bar CB represents a relation between the display color and the influence degree.

When the color bar CB is displayed, the user can visually understand the relation between the display color and the influence degree.

The display control function 458 may also cause the display 42 to display the estimation result obtained by the estimation function 456 together with the X-ray CT image data (grounds for estimation) representing the influence degree of each element.

In a case in which a plurality of types of texture feature quantities are calculated by the first calculation function 455, the display control function 458 may cause the display 42 to display the X-ray CT image data representing the influence degree of each element for each type of the texture feature. The display control function 458 may also cause the display 42 to display the X-ray CT image data representing the influence degree of each element for one type of the texture feature selected by the user.

In a case in which the contribution region is specified by the second calculation function 457, the display control function 458 may cause the display 42 to display the X-ray CT image data in which the contribution region on the X-ray CT image data is emphasized (for example, displayed in red).

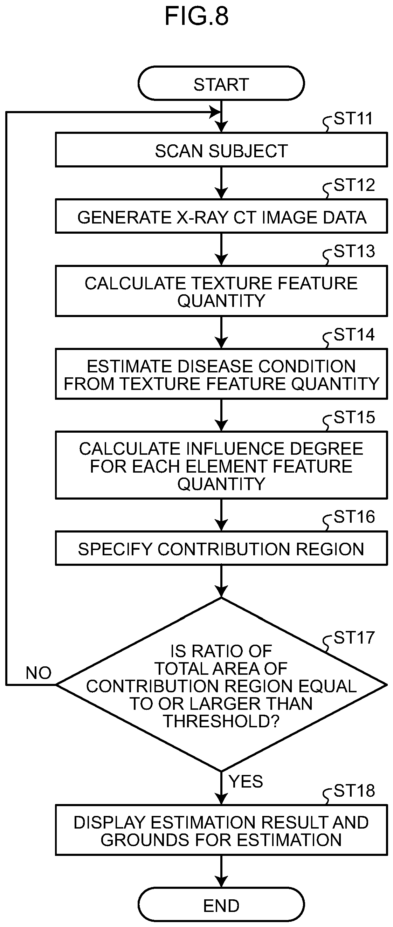

Next, the following describes processing performed by the X-ray CT apparatus 1 according to the present embodiment configured as described above. FIG. 8 is a flowchart illustrating an example of the entire procedure of the processing performed by the X-ray CT apparatus 1 according to the first embodiment.

First, the system control function 451 controls an operation of each part of the X-ray CT apparatus 1 to scan the subject P (Step ST11). For example, the system control function 451 and the preprocessing function 452 control the stand apparatus 10 and the couch apparatus 30 in accordance with the photographing protocol received from the user, irradiate the subject P with X-rays, and collect projection data from detection data of the X-rays transmitted through the subject P.

Subsequently, the reconstruction processing function 453 generates X-ray CT image data (Step ST12). For example, the reconstruction processing function 453 performs reconstruction processing on the projection data collected at Step ST11, and generates a plurality of pieces of slice image data. The image processing function 454 converts the pieces of slice image data generated by the reconstruction processing function 453 into tomogram data of an optionally cross section or three-dimensional image data using a well-known method, and generates the X-ray CT image data.

Subsequently, the first calculation function 455 calculates the texture feature quantity (Step ST13). For example, the first calculation function 455 calculates the texture feature quantity based on the X-ray CT image data generated at Step ST12 and a calculation method determined in advance for each type of the texture feature.

Subsequently, the estimation function 456 estimates the disease condition of the subject (Step ST14). For example, the estimation function 456 inputs the texture feature quantity calculated at Step ST13 to the disease condition estimation model 411 stored in the memory 41, and estimates the disease condition of the subject corresponding to the X-ray CT image data generated at Step ST12 based on an output from the disease condition estimation model 411.

Subsequently, the second calculation function 457 calculates the influence degree of each element of the texture feature (Step ST15). For example, the second calculation function 457 calculates the influence degree of each element of the texture feature by dividing each element feature quantity calculated in a process of calculating the texture feature quantity at Step ST13 by the texture feature quantity.

Subsequently, the second calculation function 457 specifies the contribution region (Step ST16). For example, the second calculation function 457 sorts the influence degrees calculated at Step ST15 in ascending order, and specifies, as the contribution region, a region on the X-ray CT image data corresponding to the element the calculated influence degree of which is the highest.

Subsequently, the system control function 451 determines whether a ratio of a total area of the contribution region to an area of the entire X-ray CT image data is equal to or larger than a predetermined threshold (Step ST17). If the ratio of the total area of the contribution region to the area of the entire X-ray CT image data is smaller than the threshold (No at Step ST17), the process returns to Step ST11.

On the other hand, if the ratio of the total area of the contribution region to the area of the entire X-ray CT image data is equal to or larger than the threshold (Yes at Step ST17), the display control function 458 performs control for displaying an estimation result of the disease condition and grounds for estimation of the estimation result (Step ST18), and ends this processing. For example, the display control function 458 causes the display 42 to display the estimation result of the disease condition at Step ST14 and the X-ray CT image data representing the contribution region specified at Step ST16.

In this way, the X-ray CT apparatus 1 according to the present embodiment generates the X-ray CT image data, calculates the texture feature quantity as one of the Radiomics feature quantities of the X-ray CT image data, specifies the contribution region indicating a region on the X-ray CT image data delineating a feature having a high influence degree for the calculation result of the texture feature quantity, and causes the X-ray CT image data representing the contribution region to be displayed.

Typically, it is difficult for even health professionals such as a doctor to understand what the value of the texture feature quantity means. On the other hand, the X-ray CT apparatus 1 according to the present embodiment can visualize a region on the X-ray CT image data that is expected to be related to the calculated value of the texture feature quantity by specifying and displaying the contribution region on the X-ray CT image data. That is, the X-ray CT apparatus 1 according to the present embodiment can efficiently visualize a region on a medical image having a high influence degree for the calculation result of the Radiomics feature quantity.

The X-ray CT apparatus 1 according to the present embodiment estimates the disease condition of the subject based on the texture feature quantity and the disease condition estimation model 411 as a pre-trained model using the texture feature quantity as an input, and causes the X-ray CT image data representing the contribution region to be displayed as the grounds for estimation of the estimation result.

In a case of estimating the disease condition using the pre-trained model that uses the texture feature quantity as an input, an appearance frequency of the texture feature on the medical image may be calculated, and information representing a region corresponding to a feature that appears on the medical image with high frequency may be presented as the grounds for estimation to the user. However, even the feature that appears with high frequency may have little relation to the estimated symptom.

On the other hand, the X-ray CT apparatus 1 according to the present embodiment presents, to the user, information representing a region on the X-ray CT image data corresponding to a feature having large influence on the calculation result of the texture feature quantity as the grounds for estimation. The pre-trained model using the texture feature quantity as an input makes an inference based on the value of the texture feature quantity, so that the feature having large influence on the calculation result of the texture feature quantity may have large influence on an output result of a trained model with high possibility.

Thus, in a case of estimating the disease condition using the pre-trained model that uses the texture feature quantity as an input, it can be considered that the X-ray CT apparatus 1 according to the present embodiment can present more appropriate information as the grounds for estimation to the user.

Second embodiment

The first embodiment has described a form in which the first calculation function 455, the estimation function 456, the second calculation function 457, and the display control function 458 are implemented by the processing circuitry of a medical image diagnosis apparatus (X-ray CT apparatus 1). A second embodiment describes a form in which functions corresponding thereto are implemented by processing circuitry of a medical image processing apparatus that is disposed separately from the medical image diagnosis apparatus.

The following mainly describes differences from the embodiment described above, and points common to the content described above will not be described in detail. Embodiments described below may be individually implemented, or may be appropriately combined with each other to be implemented.

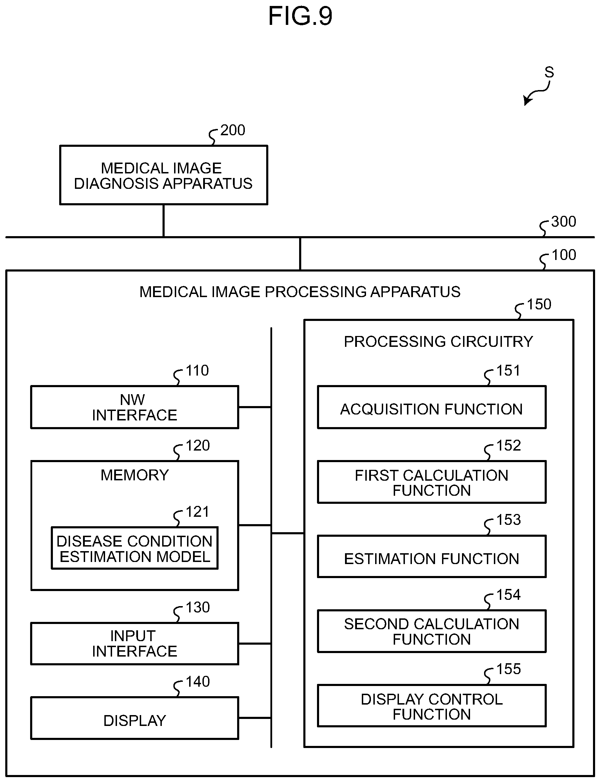

FIG. 9 is a diagram illustrating an example of the entire configuration of a medical image processing system S according to the first embodiment. As illustrated in FIG. 9, the medical image processing system S includes a medical image processing apparatus 100 and a medical image diagnosis apparatus 200, as an example. The medical image processing system S is, for example, disposed in a medical institution such as a hospital.

The medical image diagnosis apparatus 200 is an apparatus for photographing a medical image of the subject. For example, the medical image diagnosis apparatus 200 is an X-ray CT apparatus, an X-ray diagnosis apparatus, an ultrasonic diagnosis apparatus, a positron emission tomography (PET) apparatus, a magnetic resonance imaging (MRI) apparatus, a single photon emission computed tomography (SPECT) apparatus, or the like, but not limited thereto. The medical image diagnosis apparatus 200 may also be called a modality.

FIG. 9 illustrates the one medical image diagnosis apparatus 200, but a plurality of the medical image diagnosis apparatuses 200 may be disposed. Hereinafter, for convenience of explanation, the present embodiment describes a case in which the medical image diagnosis apparatus 200 is an X-ray CT apparatus, as an example.

The medical image processing apparatus 100 is, for example, a computer such as a server apparatus or a personal computer (PC). The medical image processing apparatus 100 and the medical image diagnosis apparatus 200 are connected in a communicable manner via a network 300 such as an in-hospital local area network (LAN).

The medical image processing apparatus 100 includes a network (NW) interface 110, a memory 120, an input interface 130, a display 140, and processing circuitry 150.

The NW interface 110 is connected to the processing circuitry 150, and controls communication and transmission of various kinds of data between the medical image processing apparatus 100 and the medical image diagnosis apparatus 200. The NW interface 110 is implemented by a network card, a network adapter, a network interface controller (NIC), or the like.

The memory 120 previously stores therein various pieces of information used by the processing circuitry 150. The memory 120 also stores therein various computer programs.

The memory 120 is, for example, a storage apparatus such as a hard disk drive (HDD), a solid state drive (SSD), or an integrated circuit storage apparatus that stores therein various pieces of information. In addition to the HDD, the SSD, and the like, the memory 120 may be a portable storage medium such as a compact disc (CD), a digital versatile disc (DVD), and a flash memory, or a drive apparatus that reads/writes various pieces of information from/into a semiconductor memory element such as a random access memory (RAM).

For example, the memory 120 stores therein a disease condition estimation model 121. The disease condition estimation model 121 is similar to the disease condition estimation model 411 in FIG. 1, so that the description thereof will not be repeated.

The input interface 130 is implemented by a pen tablet (drawing tablet) obtained by combining a tablet with a touch pen for receiving an operation by the user, a trackball, a switch button, a mouse, a keyboard, a touch pad for performing input operation by touching an operation surface, a touch screen obtained by integrating a display screen with a touch pad, non-contact input circuitry using an optical sensor, voice input circuitry, or the like.

The input interface 130 may include a plurality of apparatuses that receive an operation by the user. The input interface 130 is connected to the processing circuitry 150, and converts an input operation received from the user into an electric signal to be output to the processing circuitry 150.

In the present specification, the input interface does not necessarily include a physical operation component such as a mouse and a keyboard. For example, examples of the input interface include electric signal processing circuitry that receives an electric signal corresponding to the input operation from external input equipment that is disposed separately from the apparatus, and outputs the electric signal to the processing circuitry 150.

The display 140 displays various pieces of information under control by the processing circuitry 150. For example, the display 140 outputs a radiogram interpretation viewer including a medical image generated by the processing circuitry 150, a graphical user interface (GUI) for receiving various operations from the user, and the like.

Specifically, the display 140 is a liquid crystal display, a cathode ray tube (CRT) display, and the like. The input interface 130 and the display 140 may be integrated with each other. For example, the input interface 130 and the display 140 may be implemented by a touch panel.

The processing circuitry 150 is a processor that implements a function corresponding to each computer program by reading, from the memory 120, and executing the computer program. The processing circuitry 150 according to the present embodiment includes an acquisition function 151, a first calculation function 152, an estimation function 153, a second calculation function 154, and a display control function 155.

The acquisition function 151 is an example of an acquiring unit. The first calculation function 152 is an example of a first calculation unit and a second calculation unit. The estimation function 153 is an example of an estimating unit. The second calculation function 154 is an example of a second calculation unit and a specifying unit. The display control function 155 is an example of a display control unit.

The acquisition function 151 acquires medical image data obtained by photographing the subject from the medical image diagnosis apparatus 200 via the network 300 and the NW interface 110. The medical image data is, for example, the X-ray CT image data. The medical image data is, for example, two-dimensional image data or three-dimensional image data (volume data).

An acquisition source of the medical image data by the acquisition function 151 is not limited to the medical image diagnosis apparatus 200, but may be another server apparatus or the memory 120 of the medical image processing apparatus 100.

The first calculation function 152, the estimation function 153, the second calculation function 154, and the display control function 155 are the same as the first calculation function 455, the estimation function 456, the second calculation function 457, and the display control function 458 in FIG. 1 except that the medical image data acquired by the acquisition function 151 is caused to be a target of each piece of processing, so that the description thereof will not be repeated.

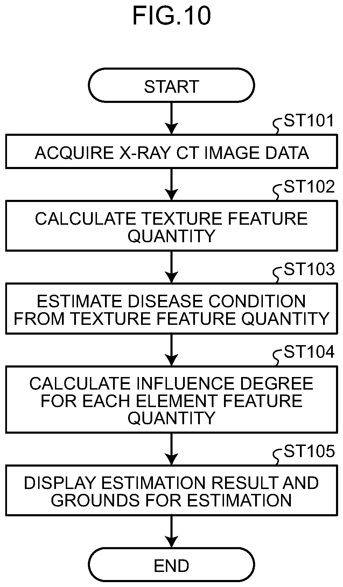

Next, the following describes processing performed by the medical image processing apparatus 100 according to the present embodiment configured as described above. FIG. 10 is a flowchart illustrating an example of the entire procedure of the processing performed by the medical image processing apparatus 100 according to the second embodiment.

First, the acquisition function 151 acquires the X-ray CT image data (Step ST101). For example, the acquisition function 151 acquires X-ray CT image data from the medical image diagnosis apparatus (X-ray CT apparatus) 200 via the NW interface 110.

The pieces of processing at Steps ST102 to Step ST104 are the same as the pieces of processing at Steps ST13 to ST15 in FIG. 8, so that description thereof will not be repeated.

After the processing at Step S104, the display control function 155 performs control for displaying the estimation result of the disease condition and the grounds for estimation of the estimation result (Step ST105), and ends this processing. For example, the display control function 155 causes the display 140 to display the estimation result of the disease condition at Step ST103 and the X-ray CT image data representing the influence degree of each element calculated at Step ST104.

With the medical image processing apparatus 100 according to the second embodiment described above, similarly to the X-ray CT apparatus 1 according to the first embodiment, a region on the medical image having a high influence degree for the calculation result of the Radiomics feature quantity can be efficiently visualized.

The first embodiment and the second embodiment described above can be appropriately modified and implemented by changing part of the configuration or functions of the X-ray CT apparatus 1 or the medical image processing apparatus 100. Thus, the following describes, as other embodiments, some modifications related to the embodiments described above.

The following mainly describes differences from the embodiments described above, and points common to the content described above will not be described in detail. The modifications described below may be individually implemented, or may be appropriately combined with each other to be implemented.


First modification

The second embodiment has described a form in which, in a case of calculating a plurality of types of texture feature quantities, the X-ray CT image data representing the influence degree of each element is displayed for each type of the texture feature. The present modification describes a form of representing the influence degree of each element of a plurality of types of feature quantities with one X-ray CT image data.

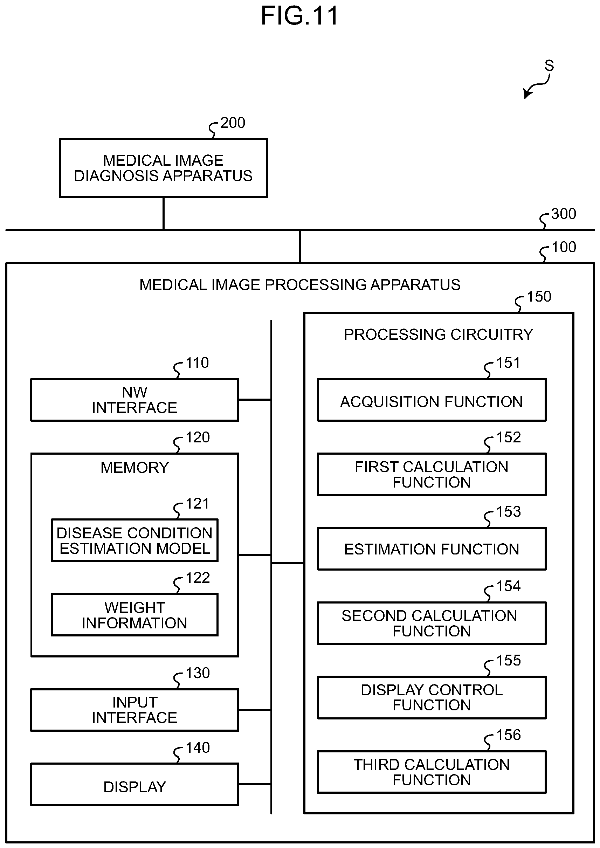

First, the following describes the configuration of the medical image processing apparatus 100 according to the present modification. FIG. 11 is a block diagram illustrating an example of the configuration of the medical image processing apparatus 100 according to a first modification. The configuration of the medical image processing apparatus 100 according to the present modification is substantially the same as the configuration of the medical image processing apparatus 100 illustrated in FIG. 9, but is different from FIG. 9 in that the memory 120 stores therein weight information 122, and the processing circuitry 150 includes a third calculation function 156.

First, the following describes the weight information 122. The weight information 122 is information defining, for the disease condition estimation model 121 that outputs the estimation result of the disease condition in accordance with inputs of a plurality of types of texture feature quantities, a weight representing how much weight is given to the type of the texture feature in estimating the disease condition for each type of the texture feature. In a case in which a plurality of the disease condition estimation models 121 are stored, the weight may be defined for each of the models in the weight information 122.

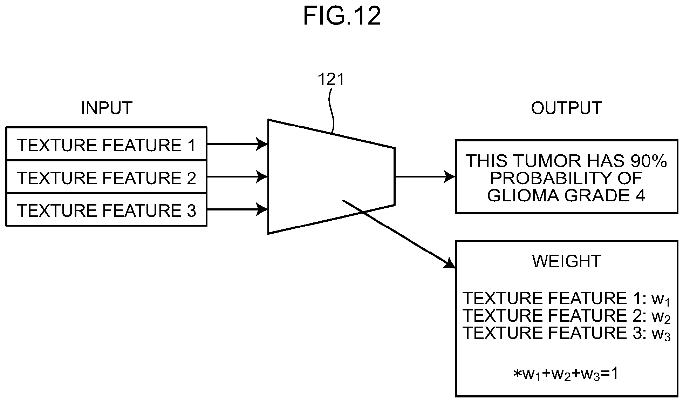

FIG. 12 is a diagram for explaining an example of the weight of the disease condition estimation model 121 according to the first modification. The disease condition estimation model 121 in the example of FIG. 12 is a pre-trained model that outputs the estimation result of the disease condition in accordance with inputs of a texture feature quantity of a texture feature 1, a texture feature quantity of a texture feature 2, and a texture feature quantity of a texture feature 3.

In the example of FIG. 12, the disease condition estimation model 121 outputs a sentence of "This tumor has a 90% probability of glioma grade 4". The example of FIG. 12 represents the fact that the weight of the texture feature 1 is $w_1$, the weight of the texture feature 2 is $w_2$, and the weight of the texture feature 3 is $w_3$.

In the above case, $w_1$ represents how much weight is given to the texture feature quantity of the texture feature 1 in outputting the estimation result of the disease condition by the disease condition estimation model 121. Similarly, $w_2$ represents how much weight is given to the texture feature quantity of the texture feature 2 in outputting the estimation result of the disease condition by the disease condition estimation model 121. Similarly, $w_3$ represents how much weight is given to the texture feature quantity of the texture feature 3 in outputting the estimation result of the disease condition by the disease condition estimation model 121.

Specifically, $w_1$, $w_2$, and $w_3$ are positive values smaller than 1, and the values of $w_1$, $w_2$, and $w_3$ are determined to satisfy $w_1 + w_2 + w_3 = 1$.

Herein, the type of the texture feature having a larger weight value has larger influence on the output result. That is, if $w_1 > w_2 > w_3$ is satisfied in the example of FIG. 12, influence on the output result of the disease condition estimation model 121 is increased in order of the texture feature quantity of the texture feature 1, the texture feature quantity of the texture feature 2, and the texture feature quantity of the texture feature 3.

Next, the following describes the third calculation function 156 of the processing circuitry 150. The third calculation function 156 is an example of a third calculation unit. The third calculation function 156 calculates an integrated influence degree obtained by integrating influence degrees of a plurality of types of the texture feature quantities. The integrated influence degree is an example of a second influence degree. The integrated influence degree represents a degree of influence given to the calculation result of a plurality of types of the texture feature quantities for each pixel on the medical image.

For example, the third calculation function 156 refers to the weight information 122 stored in the memory 120, and specifies a weight of each of the types of texture features for the disease condition estimation model 121 used for estimating the disease condition. Next, the third calculation function 156 calculates, for each type of the texture feature, a multiplication value obtained by multiplying the specified weight of the type of the texture feature by an influence degree corresponding to a certain pixel on the X-ray CT image data.

The third calculation function 156 then calculates the integrated influence degree of the certain pixel on the X-ray CT image data by totaling multiplication values of all types of the texture features. The third calculation function 156 performs the same processing on all of the pixels on the X-ray CT image data, and calculates the integrated influence degree for each of the pixels on the X-ray CT image data.

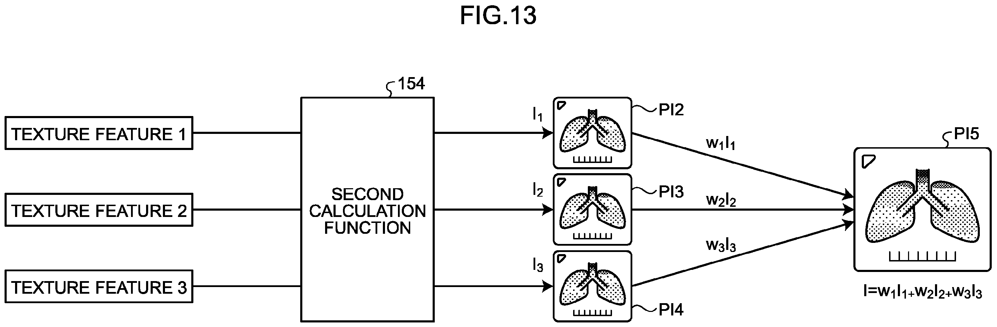

FIG. 13 is a diagram for explaining an example of calculation processing for the integrated influence degree according to the first modification. FIG. 13 represents an example of the calculation method for the integrated influence degree in a case of using the disease condition estimation model 121 in FIG. 12 for estimating the disease condition.

A processed image PI2 in FIG. 13 is X-ray CT image data obtained by changing a display color of each pixel in accordance with the influence degree of the texture feature 1. A processed image PI3 is X-ray CT image data obtained by changing a display color of each pixel in accordance with the influence degree of the texture feature 2. A processed image PI4 is X-ray CT image data obtained by changing a display color of each pixel in accordance with the influence degree of the texture feature 3.

An influence degree $I_1$ in FIG. 13 represents an influence degree of the texture feature 1 corresponding to a certain pixel on the X-ray CT image data calculated by the second calculation function 154. Similarly, an influence degree $I_2$ represents an influence degree of the texture feature 2 corresponding to a certain pixel on the X-ray CT image data. Similarly, an influence degree $I_3$ represents an influence degree of the texture feature 3 corresponding to a certain pixel on the X-ray CT image data.

In the example of FIG. 13, the third calculation function 156 calculates an integrated influence degree $I$ of a certain pixel on the X-ray CT image data by totaling $w_1 I_1$ obtained by multiplying the weight $w_1$ of the texture feature 1 by the influence degree $I_1$, $w_2 I_2$ obtained by multiplying the weight $w_2$ of the texture feature 2 by the influence degree $I_2$, and $w_3 I_3$ obtained by multiplying the weight $w_3$ of the texture feature 3 by the influence degree $I_3$.

Due to this, the display control function 155 can cause the display 140 to display a processed image PI5 as the X-ray CT image data in which the display color of each pixel is changed in accordance with the integrated influence degree calculated by the third calculation function 156.

The third calculation function 156 may specify a common contribution region indicating a region on the X-ray CT image data delineating a feature having a high influence degree for all calculation results of a plurality of types of the texture feature quantities.

In this case, the third calculation function 156 may specify the contribution region for each of a plurality of types of the texture feature quantities, and specify, as the common contribution region, a region on the X-ray CT image data in which all of the specified contribution regions overlap each other.

The display control function 155 according to the present modification causes the display 140 to display the X-ray CT image data representing the integrated influence degree of each pixel on the X-ray CT image data calculated by the third calculation function 156.

In a case in which the common contribution region is specified by the third calculation function 156, the display control function 155 may cause the display 140 to display the X-ray CT image data in which the common contribution region on the X-ray CT image data is emphasized.

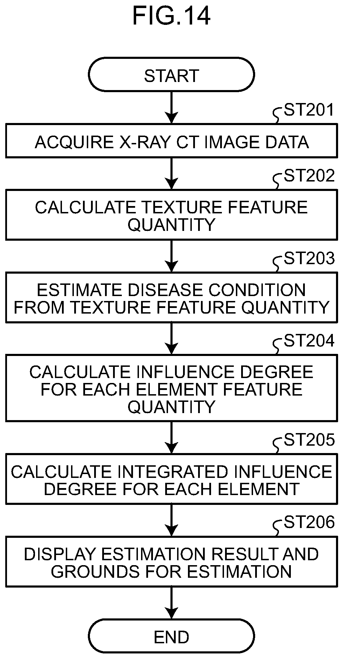

Next, the following describes processing performed by the medical image processing apparatus 100 according to the present modification. FIG. 14 is a flowchart illustrating an example of the processing performed by the medical image processing apparatus 100 according to the first modification.

The pieces of processing at Step ST201 to Step ST204 in FIG. 14 are substantially the same as the pieces of processing at Step ST101 to Step ST104 in FIG. 10, but in FIG. 14, a plurality of types of the texture feature quantities are calculated at Step ST202. At Step ST203, a plurality of types of the texture feature quantities are input to the disease condition estimation model 121. At Step ST204, the influence degree is calculated for each of a plurality of types of the texture features.

After Step ST204, the third calculation function 156 calculates the integrated influence degree (Step ST205).

For example, the third calculation function 156 refers to the weight information 122 stored in the memory 120, and specifies a weight of each of a plurality of types of the texture features for the disease condition estimation model 121 used at Step ST203.

The third calculation function 156 calculates, for each type of the texture feature, a multiplication value obtained by multiplying the specified weight by the influence degree calculated at Step ST203 corresponding to a certain pixel on the X-ray CT image data. The third calculation function 156 calculates the integrated influence degree of the certain pixel on the X-ray CT image data by totaling multiplication values. The third calculation function 156 performs the same processing on all of the pixels on the X-ray CT image data, and calculates the integrated influence degree for each of the pixels on the X-ray CT image data.

Subsequently, the display control function 155 performs control for displaying the estimation result of the disease condition and the grounds for estimation of the estimation result (Step ST206), and ends this processing. For example, the display control function 155 causes the display 140 to display the estimation result of the disease condition at Step ST203 and the X-ray CT image data representing the integrated influence degree of each pixel on the X-ray CT image data calculated at Step ST205.

The X-ray CT apparatus 1 may be configured so that the X-ray CT apparatus 1 according to the first embodiment can perform the processing according to the present modification.

With the present modification, even in a case of estimating the disease condition using a plurality of types of the texture feature quantities, a region on the medical image having large influence on estimation of the disease condition can be visualized.

Second modification

The second embodiment has described a form of displaying the estimation result of the disease condition and the X-ray CT image data as the grounds for estimation. The present modification describes a form of further displaying a reference image together therewith.

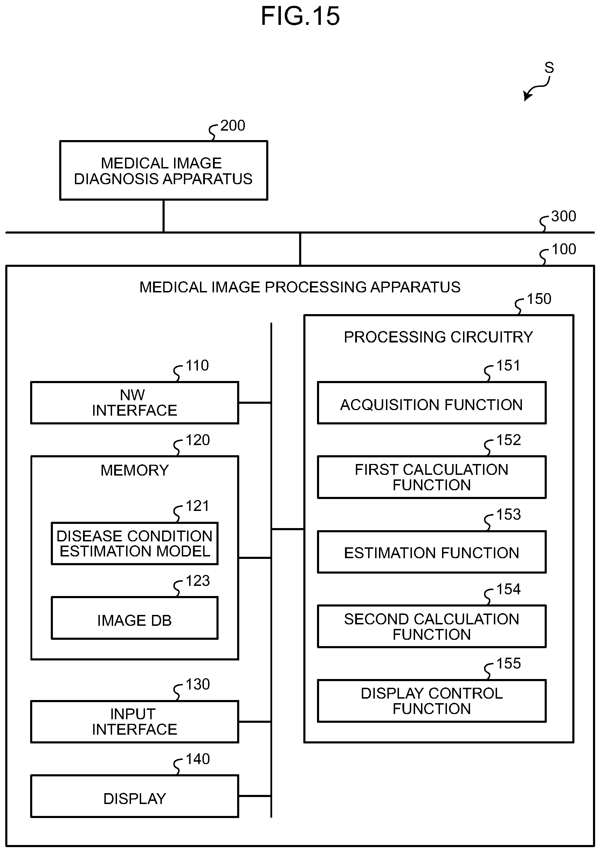

First, the following describes the configuration of the medical image processing apparatus 100 according to the present modification. FIG. 15 is a block diagram illustrating an example of the configuration of the medical image processing apparatus 100 according to a second modification. The configuration of the medical image processing apparatus 100 according to the present modification is substantially the same as the configuration of the medical image processing apparatus 100 illustrated in FIG. 9, but is different from FIG. 9 in that the memory 120 stores therein an image DB 123.

First, the following describes the image database (DB) 123. The image DB 123 is a database that stores therein various medical images.

For example, the image DB 123 stores therein a symptom (for example, "glioma grade 4" and the like) and a medical image representing a typical example of the symptom in association with each other. For example, the image DB 123 stores therein a medical image and a value of a texture feature quantity of the medical image in association with each other. For example, the image DB 123 stores therein a plurality of medical images representing the influence degree of each element.

At the time of causing the display 140 to display a medical image as the grounds for estimation, the display control function 155 according to the present modification causes the display 140 to display a reference image together therewith.

For example, in a case of receiving an instruction to display a typical image (hereinafter, also referred to as a typical example) of an estimated symptom as a reference image from the user, the display control function 155 refers to the image DB 123, specifies the X-ray CT image data corresponding to the symptom estimated by the estimation function 153, and causes the display 140 to display the specified X-ray CT image data as the reference image together with the medical image as the grounds for estimation.

For example, in a case of receiving an instruction to display an image having a similar value of the texture feature quantity as the reference image from the user, the display control function 155 refers to the image DB 123, specifies X-ray CT image data corresponding to a value of the texture feature quantity on the image DB 123 closest to the value of the texture feature quantity calculated by the first calculation function 152, and causes the display 140 to display the specified X-ray CT image data as the reference image together with the medical image as the grounds for estimation.

For example, in a case of receiving an instruction to display an image with similar distribution of contribution regions as the reference image from the user, the display control function 155 searches for X-ray CT image data representing the influence degree of each element of the image DB 123. The display control function 155 then causes the display 140 to display, as the reference image, X-ray CT image data having the highest similarity to the X-ray CT image data as the grounds for estimation together with the X-ray CT image data as the grounds for estimation.

In the above description, described is an example of causing the display 140 to display, in accordance with the instruction from the user, any of the typical example, the image having a similar value of the texture feature quantity, and the image with similar distribution of contribution regions as the reference image, but the method of displaying the reference image is not limited thereto.

For example, the display control function 155 may cause the display 140 to display, as reference images, all of the typical example, the image having a similar value of the texture feature quantity, and the image with similar distribution of contribution regions. For example, the display control function 155 may cause the display 140 to display, as reference images, one or a plurality of images previously set by the user among the typical example, the image having a similar value of the texture feature quantity, and the image with similar distribution of contribution regions.

A type of the image as the reference image is not limited to the typical example, the image having a similar value of the texture feature quantity, and the image with similar distribution of contribution regions. For example, a normal image corresponding to an age, a gender, and the like of the subject may be used as the reference image.

The display control function 155 may also cause the display 140 to display an estimation result obtained by the estimation function 153 in addition to the reference image and the X-ray CT image data as the grounds for estimation.

The X-ray CT apparatus 1 may be configured so that the X-ray CT apparatus 1 according to the first embodiment can perform the processing according to the present modification.

According to the present modification, for example, the user can check the reference image of a type corresponding to a situation together with the medical image as the grounds for estimation, so that validity of the grounds for estimation can be easily performed.

Third modification

The first embodiment and the second embodiment have described an example of applying this method to the Joint Energy feature quantity and the SAE feature quantity, but the feature quantity to which this method can be applied is not limited thereto. For example, this method can be applied to a feature quantity corresponding to any of the following expressions (5) to (8).

$$Texture\ feature\ quantity = \sum_{i=1}^{N_g} \sum_{j=1}^{N_g} (Function\ of\ pixel\ values\ i, j)$$

$$\cdots (5)$$

$$\mathit{Texture\ feature\ quantity} =$$

$$\sum\nolimits_{k=0}^{N_g-1} \sum\nolimits_{i=1}^{N_g} \sum\nolimits_{j=1}^{N_g} (\mathit{Function\ of\ pixel\ values\ i, j}) \qquad \cdots (6)$$

where$|i - j| = k$

$$\mathit{Texture\ feature\ quantity} =$$

$$\sum\nolimits_{k=2}^{2N_g} \sum\nolimits_{i=1}^{N_g} \sum\nolimits_{j=1}^{N_g} (\mathit{Function\ of\ pixel\ values\ i, j}) \qquad \cdots (7)$$

where $i + j = k$

$$\mathit{Texture\ feature\ quantity} = \max(\mathit{Function\ of\ pixel\ values\ i, j}) \quad \cdots (8)$$

Fourth modification

In the second embodiment described above, the medical image processing apparatus 100 acquires the medical image data from the medical image diagnosis apparatus 200, but an acquisition source of the medical image data is not limited thereto. The medical image processing apparatus 100 may acquire the medical image data from a medical image storage apparatus that stores medical images photographed by the medical image diagnosis apparatus 200.
The medical image storage apparatus is, for example, a server apparatus of a picture archiving and communication system (PACS), and stores the medical image data in a format conforming to digital imaging and communications in medicine (DICOM) .

Fifth modification

The first embodiment and the second embodiment have described a form of performing a series of processing on the medical image as a target. However, the image as the target of this method is not limited to the medical image. For example, this method can be applied to an image delineating a plant, an animal, an article, and the like.
For example, in a case of using an image of a plant, the estimation function 456 (153) may perform processing of estimating a name (type) of the plant delineated in the image. In this case, the estimation function 456 (153) may perform processing of estimating the name of the plant by inputting a calculated texture feature quantity to a pre-trained model that outputs the name of the plant in accordance with an input of a texture feature quantity trained by a well-known machine learning technique.
For example, in a case of using an image of an animal, the estimation function 456 (153) may perform processing of estimating a name (type) of the animal delineated in the image. In this case, the estimation function 456 (153) may perform processing of estimating the name of the animal by inputting a calculated texture feature quantity to a pre-trained model that outputs the name of the animal in accordance with an input of a texture feature quantity trained by a well-known machine learning technique.
For example, in a case of using an image of an article, the estimation function 456 (153) may perform processing of estimating a name (type) of the article delineated in the image. In this case, the estimation function 456 (153) may perform processing of estimating the name of the article by inputting a calculated texture feature quantity to a pre-trained model that outputs the name of the article in accordance with an input of a texture feature quantity trained by a well-known machine learning technique.
According to the present modification, even in a case in which an image other than the medical image is the target, a region on an image having a high influence degree for the calculation result of the texture feature quantity can be efficiently visualized.
Various kinds of data treated in the present specification are typically digital data.
A word of "processor" used in the above description means, for example, a central processing unit (CPU), a graphics processing unit (GPU), or circuitry such as an application specific Integrated circuit (ASIC) and a programmable logic device (for example, a simple programmable logic device (SPLD), a complex programmable logic device (CPLD), and a field programmable gate array (FPGA)).
Herein, instead of storing a computer program in the memory, the computer program may be directly incorporated in circuitry of the processor. In this case, the processor implements a function by reading out and executing the computer program incorporated in the circuitry. Each of the processors in the first embodiment and the second embodiment described above is not necessarily configured as a single piece of circuitry for each processor. A plurality of independent pieces of circuitry may be combined to configure one processor to implement the function thereof.

According to at least one of the embodiments described above, a region on a medical image having a high influence degree for a calculation result of the Radiomics feature quantity can be efficiently visualized.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims. The accompanying claims are intended to cover such forms or modifications as would fall within the scope of the inventions.

**Claims**

1. An X-ray CT apparatus (1) comprising:

   a photographing system (19) configured to photograph a subject;
   a generation unit (453) configured to generate X-ray CT image data by performing reconstruction processing on projection data output from the photographing system (19);
   a first calculation unit (455) configured to calculate an image feature quantity indicating a feature of a relation between one pixel on the X-ray CT image data and one or a plurality of pixels other than the former pixel;
   a specifying unit (457) configured to specify a contribution region indicating a region on the X-ray CT image data delineating the feature having a high influence degree for a calculation result of the image feature quantity; and
   a display control unit (458) configured to cause a display apparatus to display the X-ray CT image data representing the contribution region.

2. The X-ray CT apparatus (1) according to claim 1, further comprising:

   a second calculation unit (457) configured to

      calculate an element feature quantity as a feature quantity corresponding to each of features on the X-ray CT image data for each of the features, and
      calculate a first influence degree indicating a degree of influence of the feature on a calculation result of the image feature quantity based on the image

   feature quantity and the element feature quantity, 14wherein
   the specifying unit (457) is configured to specify the contribution region based on the first influence degree.

3. The X-ray CT apparatus (1) according to claim 2, wherein
   the specifying unit (457) is configured to specify, as the contribution region, a region corresponding to the feature having a highest first influence degree on the X-ray CT image data.

4. The X-ray CT apparatus (1) according to claim 2, wherein
   the specifying unit (457) is configured to specify, as the contribution region, a region corresponding to the feature having the first influence degree exceeding a threshold on the X-ray CT image data.

5. The X-ray CT apparatus (1) according to claim 2, further comprising:

   an estimating unit (456) configured to estimate a disease condition of the subject based on the image feature quantity and a pre-trained model (411) caused to have a function of outputting an estimation result of a disease condition in response to an input of the image feature quantity by machine learning, wherein
   the display control unit (458) is configured to cause the display apparatus (42) to display the X-ray CT image data representing the contribution region as grounds for estimation of the estimation result.

6. The X-ray CT apparatus (1) according to claim 5, wherein

the first calculation unit (455) is configured to calculate a plurality of types of the image feature quantities, the second calculation unit (457) is configured to calculate the first influence degree for each of a plurality of types of the image feature quantities, the apparatus further comprising:
a third calculation unit (156) configured to

calculate the first influence degree for each of a plurality of types of the image feature quantities, and calculate, for each pixel on the X-ray CT image data, a second influence degree indicating a degree of influence of the pixel on each of a plurality of types of the image feature quantities based on the first influence degree and a weight indicating how much weight is given to a specific type of the image feature quantity when the pre-trained model outputs the estimation result, wherein

the specifying unit (457) is configured to specify the contribution region based on the second influence degree.

7. The X-ray CT apparatus (1) according to claim 1, wherein

the specifying unit (457) is configured to specify, based on the specified contribution region and an image database storing therein a plurality of pieces of the X-ray CT image data representing the contribution region, the X-ray CT image data with similar distribution of contribution regions on the X-ray CT image data in the image database, and
the display control unit (458) is configured to cause the specified X-ray CT image data to be displayed as a reference image.

8. A medical image processing apparatus (300) comprising:

an acquiring unit (151) configured to acquire a medical image;
a first calculation unit (152) configured to calculate an image feature quantity indicating a feature of a relation between one pixel on the medical image and one or a plurality of pixels other than the former pixel;
a specifying unit (154) configured to specify a contribution region indicating a region on the medical image delineating the feature having a high influence degree for a calculation result of the image feature quantity; and
a display control unit (155) configured to cause a display apparatus (140) to display the medical image representing the contribution region.

9. The medical image processing apparatus (300) according to claim 8, further comprising:

a second calculation unit (154) configured to

calculate an element feature quantity as a feature quantity corresponding to each of features on the medical image for each of the features, and
calculate a first influence degree indicating a degree of influence of the feature on a calculation result of the image feature quantity based on the image feature quantity and the element feature quantity,

wherein
the specifying unit (154) is configured to specify the contribution region based on the first influence degree.

10. The medical image processing apparatus (300) according to claim 9, wherein
the specifying unit (154) is configured to specify, as the contribution region, a region corresponding to the feature having a highest first influence degree on the medical image.

11. The medical image processing apparatus (300) according to claim 9, wherein
the specifying unit (154) is configured to specify, as the contribution region, a region corresponding to the feature having the first influence degree exceeding a threshold on the medical image.

12. The medical image processing apparatus (300) according to claim 9, further comprising:

an estimating unit (153) configured to estimate a disease condition of the subject based on the image feature quantity and a pre-trained model (121) caused to have a function of outputting an estimation result of a disease condition in response to an input of the image feature quantity by machine learning, wherein
the display control unit (155) is configured to cause the display apparatus (140) to display the medical image representing the contribution region as grounds for estimation of the estimation result.

13. The medical image processing apparatus (300) according to claim 12, wherein

the first calculation unit (152) is configured to calculate a plurality of types of the image feature quantities, and the second calculation unit (154) is configured to calculate the first influence degree for each of a plurality of types of the image feature quantities, the apparatus further comprising:

a third calculation unit (156) configured to calculate, for each pixel on the medical image, a second influence degree indicating a degree of influence of the pixel on each of a plurality of types of the image feature quantities based on the first influence degree and a weight indicating how much weight is given to a specific type of the image feature quantity when the pre-trained model outputs the estimation result, wherein

the specifying unit (154) is configured to specify the contribution region based on the second influence degree.

14. The medical image processing apparatus (300) according to claim 8, wherein

the specifying unit (154) is configured to specify, based on the specified contribution region and an image database storing therein a plurality of the medical images representing the contribution region, the medical image with similar distribution of contribution regions on the medical image in the image database, and

the display control unit (155) is configured to cause the specified medical image to be displayed as a reference image.

15. A medical image processing method performed by a medical image processing apparatus, the medical image processing method comprising:

acquiring a medical image;

calculating an image feature quantity indicating a feature of a relation between one pixel on the medical image and one or a plurality of pixels other than the former pixel,

specifying a contribution region indicating a region on the medical image delineating a feature having a high influence degree for a calculation result of the image feature quantity, and

causing a display apparatus (140) to display the medical image representing the contribution region.

FIG.1

# FIG.2

```
┌─────────────┐     ┌─────────────┐     ┌─────────────┐
│   TEXTURE   │     │   DISEASE   │     │             │
│   FEATURE   │ ──▶ │  CONDITION  │ ──▶ │ ESTIMATION  │
│  QUANTITY   │     │ ESTIMATION  │     │   RESULT    │
│             │     │    MODEL    │     │             │
└─────────────┘     └─────────────┘     └─────────────┘
```

# FIG.3

# FIG.4

CONNECTION NUMBER M2

PIXEL VALUE

| 0 | 0 | 0 | 1 | 0 |
|---|---|---|---|---|
| 1 | 0 | 0 | 0 | **1** |
| 1 | 0 | 1 | 0 | 1 |
| 1 | 1 | 0 | 0 | 0 |
| 3 | 0 | 0 | 0 | 0 |

PIXEL VALUE OF MEDICAL IMAGE V2

| 5 | 2 | 5 | 4 | 4 |
|---|---|---|---|---|
| 3 | 3 | 3 | 1 | 3 |
| 2 | 1 | 1 | 1 | 3 |
| 4 | 2 | 2 | 2 | 3 |
| 3 | 5 | 3 | 3 | 2 |

# FIG.5

INFLUENCE DEGREE

| ELEMENT | $p(1,2)^2$ | $p(0,1)^2$ | ... | $p(2,4)^2$ | ... | $p(4,4)^2$ |
|---|---|---|---|---|---|---|
| INFLUENCE DEGREE OF ELEMENT (ASCENDING ORDER) | $\dfrac{W}{300}$ | $\dfrac{X}{300}$ | ... | $\dfrac{Y}{300}$ | ... | $\dfrac{Z}{300}$ |
| MEANING OF ELEMENT | AR-RANGE-MENT OF 1, 2 | AR-RANGE-MENT OF 0, 1 | ... | AR-RANGE-MENT OF 2, 4 | ... | AR-RANGE-MENT OF 4, 4 |

# FIG.6

INFLUENCE DEGREE

| ELEMENT | $\dfrac{P(2,1)}{1^2}$ | ... | $\dfrac{P(1,5)}{5^2}$ |
|---|---|---|---|
| INFLUENCE DEGREE OF ELEMENT (ASCENDING ORDER) | $\dfrac{V}{100}$ | ... | 0 |
| MEANING OF ELEMENT | REGION IN WHICH 1 PIXEL HAVING PIXEL VALUE OF 2 IS CONNECTED | ... | REGION IN WHICH 5 PIXELS HAVING PIXEL VALUE OF 1 ARE CONNECTED |

# FIG.7

PI1

CB

LARGE — INFLUENCE DEGREE — SMALL

# FIG.8

```
START
```

ST11
SCAN SUBJECT

ST12
GENERATE X-RAY CT IMAGE DATA

ST13
CALCULATE TEXTURE FEATURE
QUANTITY

ST14
ESTIMATE DISEASE CONDITION
FROM TEXTURE FEATURE QUANTITY

ST15
CALCULATE INFLUENCE DEGREE
FOR EACH ELEMENT FEATURE
QUANTITY

ST16
SPECIFY CONTRIBUTION REGION

ST17
IS RATIO OF
TOTAL AREA OF
CONTRIBUTION REGION EQUAL
TO OR LARGER THAN
THRESHOLD?

NO

YES

ST18
DISPLAY ESTIMATION RESULT AND
GROUNDS FOR ESTIMATION

```
END
```

# FIG.9

S

MEDICAL IMAGE DIAGNOSIS APPARATUS — 200

— 300

MEDICAL IMAGE PROCESSING APPARATUS — 100

PROCESSING CIRCUITRY — 150

NW INTERFACE — 110

MEMORY — 120

DISEASE CONDITION ESTIMATION MODEL — 121

INPUT INTERFACE — 130

DISPLAY — 140

ACQUISITION FUNCTION — 151

FIRST CALCULATION FUNCTION — 152

ESTIMATION FUNCTION — 153

SECOND CALCULATION FUNCTION — 154

DISPLAY CONTROL FUNCTION — 155

# FIG.10

```
        ┌──────────────┐
        │    START     │
        └──────┬───────┘
               │          ⌇ST101
        ┌──────▼────────────────────┐
        │ ACQUIRE X-RAY CT IMAGE DATA│
        └──────┬────────────────────┘
               │          ⌇ST102
        ┌──────▼────────────────────┐
        │   CALCULATE TEXTURE FEATURE│
        │          QUANTITY          │
        └──────┬────────────────────┘
               │          ⌇ST103
        ┌──────▼────────────────────┐
        │   ESTIMATE DISEASE CONDITION│
        │ FROM TEXTURE FEATURE QUANTITY│
        └──────┬────────────────────┘
               │          ⌇ST104
        ┌──────▼────────────────────┐
        │  CALCULATE INFLUENCE DEGREE│
        │   FOR EACH ELEMENT FEATURE │
        │          QUANTITY          │
        └──────┬────────────────────┘
               │          ⌇ST105
        ┌──────▼────────────────────┐
        │  DISPLAY ESTIMATION RESULT AND│
        │    GROUNDS FOR ESTIMATION  │
        └──────┬────────────────────┘
               │
        ┌──────▼───────┐
        │     END      │
        └──────────────┘
```

# FIG.11

S

200

MEDICAL IMAGE
DIAGNOSIS APPARATUS

300

100

## MEDICAL IMAGE PROCESSING APPARATUS

150

PROCESSING CIRCUITRY

110

NW
INTERFACE

120

MEMORY

121

DISEASE CONDITION
ESTIMATION MODEL

122

WEIGHT
INFORMATION

130

INPUT
INTERFACE

140

DISPLAY

151

ACQUISITION FUNCTION

152

FIRST CALCULATION
FUNCTION

153

ESTIMATION FUNCTION

154

SECOND CALCULATION
FUNCTION

155

DISPLAY CONTROL
FUNCTION

156

THIRD CALCULATION
FUNCTION

# FIG.12

INPUT

121

OUTPUT

| TEXTURE FEATURE 1 |
| TEXTURE FEATURE 2 |
| TEXTURE FEATURE 3 |

THIS TUMOR HAS 90%
PROBABILITY OF
GLIOMA GRADE 4

WEIGHT

TEXTURE FEATURE 1: $w_1$
TEXTURE FEATURE 2: $w_2$
TEXTURE FEATURE 3: $w_3$

*$w_1 + w_2 + w_3 = 1$

FIG.13

# FIG.14

START

↓ ST201

ACQUIRE X-RAY CT IMAGE DATA

↓ ST202

CALCULATE TEXTURE FEATURE
QUANTITY

↓ ST203

ESTIMATE DISEASE CONDITION
FROM TEXTURE FEATURE QUANTITY

↓ ST204

CALCULATE INFLUENCE DEGREE
FOR EACH ELEMENT FEATURE
QUANTITY

↓ ST205

CALCULATE INTEGRATED INFLUENCE
DEGREE FOR EACH ELEMENT

↓ ST206

DISPLAY ESTIMATION RESULT AND
GROUNDS FOR ESTIMATION

↓

END

# FIG.15

S

MEDICAL IMAGE
DIAGNOSIS APPARATUS
200

300

100

MEDICAL IMAGE PROCESSING APPARATUS

150

PROCESSING CIRCUITRY

NW
INTERFACE
110

ACQUISITION FUNCTION
151

MEMORY
120

FIRST CALCULATION
FUNCTION
152

DISEASE CONDITION
ESTIMATION MODEL
121

ESTIMATION FUNCTION
153

IMAGE DB
123

SECOND CALCULATION
FUNCTION
154

INPUT
INTERFACE
130

DISPLAY CONTROL
FUNCTION
155

DISPLAY
140

**EUROPEAN SEARCH REPORT**

Application Number

EP 25 16 8351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/287667 A1 (SUZUKI YOJIRO [JP]) 15 September 2022 (2022-09-15) * Figures: 1, 6 ; [0015], [0021], [0046], [0064-0066], [0070-0079], [0085], [0109] ; * | 1-15 | INV. G06T11/00 |
| | ----- | | |
| A | ALEXANDER CAVALLARO ET AL: "Region of Interest Queries in CT Scans", 24 August 2011 (2011-08-24), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 56 - 73, XP019162170, ISBN: 978-3-540-74549-5 * figures 1,2 * | 1 | |
| | ----- | | |
| A | US 10 219 775 B2 (TOSHIBA MEDICAL SYS CORP [JP]) 5 March 2019 (2019-03-05) ----- | 1 | |

TECHNICAL FIELDS SEARCHED (IPC)

G06V
G06T

The present search report has been drawn up for all claims

| Place of search | Date of completion of the search | Examiner |
|---|---|---|
| The Hague | 18 August 2025 | Versluis, Anton |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ..................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 16 8351

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2025

| Patent document cited in search report | Publication date | Patent family member(s) | Publication date |
|---|---|---|---|
| US 2022287667 A1 | 15-09-2022 | JP 7721288 B2<br>JP 2022138816 A<br>US 2022287667 A1 | 12-08-2025<br>26-09-2022<br>15-09-2022 |
| US 10219775 B2 | 05-03-2019 | NONE | |

EPO FORM P0459

For more details about this annex : see Official Journal of the European Patent Office, No. 12/82